# EUROPÄISCHE PATENTANMELDUNG

(11) **EP 0 704 434 A1**
(43) Veröffentlichungstag der Anmeldung: **03.04.1996**
(21) Anmeldenummer: 95112760.4
(22) Anmeldetag: 14.08.1995
(51) Int. Cl.: C07D 211/92, C07D 211/90, A61K 31/44, C07D 471/02

(54) **6-Amino-1,4-Dihydropyridinen und ihre Verwendung als selektive Kaliumkanalmodulatoren**

(30) Priorität: 25.08.1994 DE 4430095
(71) Anmelder: BAYER AG, D-51368 Leverkusen (DE)
(72) Erfinder: Urbahns, Klaus, Dr., D-42115 Wuppertal (DE); Goldmann, Siegfried, Dr., D-42327 Wuppertal (DE); Heine, Hans-Georg, Dr., D-47800 Krefeld (DE); Junge, Bodo, Dr., D-42399 Wuppertal (DE); Schohe-Loop, Rudolf, Dr., D-42327 Wuppertal (DE); Sommermeyer, Henning, Dr., D-51061 Köln (DE); Glaser, Thomas, Dr., D-51491 Overath (DE); Wittka, Reilinde, Dr., D-51069 Köln (DE); de Vry, Jean-Marie-Viktor, Dr., D-51503 Rösrath (DE)

(57) **Zusammenfassung**

Die vorliegende Erfindung betrifft die neue Verwendung von 6-Amino-5-nitro- und -5-cyano-1,4-dihydropyridinen der allgemeinen Formel (I) 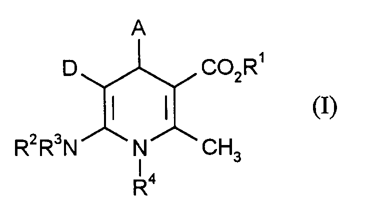
in welcher
A, D, R¹-R⁴ die in der Beschreibung angegebene Bedeutung haben, neue 6-Acylamino-dihydropyridine, Verfahren zu ihrer Herstellung und ihre Verwendung als Arzneimittel, als selektive Kaliumkanalmodulatoren, insbesondere zur Behandlung des zentralen Nervensystems.

## Beschreibung

Die vorliegende Erfindung betrifft die neue Verwendung von 6-Amino-5-nitro- und -5-cyano-1,4-dihydropyridinen, neue 6-Acylamino-dihydropyridine, Verfahren zu ihrer Herstellung und ihre Verwendung als Arzneimittel, als selektive Kaliumkanalmodulatoren, insbesondere zur Behandlung des zentralen Nervensystems.

Amino-substituierte 1,4-Dihydropyridine und ihre calciumantagonistischen Wirkungen sind bereits bekannt [vgl. hierzu Arch. Pharm. 324 (2), 73-7, 1991; Arch. Pharm. 322 (5), 285-90, 1989 und DOS 22 10 674].

Es wurde gefunden, daß die teilweise bekannten 6-Amino-5-nitro- und -5-cyano-1,4-dihydropyridine der allgemeinen Formel (I) 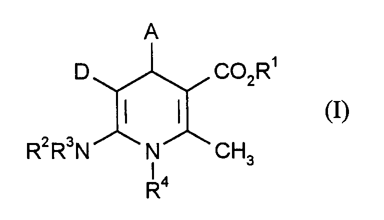
in welcher
- A: für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
- D: für Cyano oder Nitro steht,
- R¹: für Wasserstoff oder für geradkettiges oder verzweigtes Alkyl mit bis zu 8 Kohlenstoffatomen steht,
- R² und R³: gleich oder verschieden sind und
für Wasserstoff oder für geradkettiges oder verzweigtes Alkyl oder Acyl mit jeweils bis zu 6 Kohlenstoffatomen stehen,
- R⁴: für Wasserstoff steht,
oder
- R³ und R⁴: zusammen für einen Rest der Formel -(CH₂)ₙ stehen,
worin
- n: eine Zahl 2, 3 oder 4 bedeutet,
und deren physiologisch unbedenklichen Salze,
überraschenderweise eine selektive modulierende Wirkung auf Kaliumkanäle besitzen und geeignet sind zur Verwendung bei der Bekämpfung von cerebralen Erkrankungen und der Sichelzellenanämie.

Physiologisch unbedenkliche Salze sind im allgemeinen Salze der erfindungsgemäßen Verbindungen mit anorganischen oder organischen Säuren. Bevorzugt werden Salze mit anorganischen Säuren wie beispielsweise Salzsäure, Bromwasserstoffsäure, Phosphorsäure oder Schwefelsäure, oder Salze mit organischen Carbon- oder Sulfonsäuren wie beispielsweise Essigsäure, Maleinsäure, Fumarsäure, Äpfelsäure, Zitronensäure, Weinsäure, Milchsäure, Benzoesäure, oder Methansulfonsäure, Ethansulfonsäure, Phenylsulfonsäure, Toluolsulfonsäure oder Naphthalindisulfonsäure.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen.

Bevorzugt ist die Verwendung von Verbindungen der allgemeinen Formel (I),
in welcher
- A: für Phenyl, Naphthyl oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 4 Kohlenstoffatomen substituiert sind,
- D: für Cyano oder Nitro steht,
- R¹: für geradkettiges oder verzweigtes Alkyl mit bis zu 6 Kohlenstoffatomen steht,
- R² und R³: gleich oder verschieden sind und für Wasserstoff oder für geradkettiges oder verzweigtes Alkyl oder Acyl mit jeweils bis zu 4 Kohlenstoffatomen stehen,
- R⁴: für Wasserstoff steht,
oder
- R³ und R⁴: zusammen für einen Rest der Formel -(CH₂)ₙ stehen,
worin
- n: eine Zahl 2 oder 3 bedeutet,
und deren Salze.

Besonders bevorzugt ist die Verwendung von Verbindungen der allgemeinen Formel (I),
in welcher
- A: für Phenyl oder Pyridyl steht, die gegebenenfalls bis zu 2-fach gleich oder verschieden durch Nitro, Cyano, Phenyl, Fluor, Chlor, Trifluormethyl, Methoxy oder Methylthio substituiert sind,
- D: für Cyano oder Nitro steht,
- R¹: für geradkettiges oder verzweigtes Alkyl mit bis zu 4 Kohlenstoffatomen steht,
- R² und R³: gleich oder verschieden sind und für Wasserstoff oder für geradkettiges oder verzweigtes Alkyl oder Acyl mit jeweils bis zu 4 Kohlenstoffatomen stehen,
- R⁴: für Wasserstoff steht,
oder
- R³ und R⁴: zusammen für einen Rest der Formel -(CH₂)ₙ stehen,
worin
- n: eine Zahl 2 oder 3 bedeutet,
und deren Salze
bei der Bekämpfung von cerebralen Erkrankungen.

Die erfindungsgemäßen Verbindungen der allgemeinen Formel (I) zeigen ein nicht vorhersehbares, wertvolles pharmakologisches Wirkspektrum.

Sie sind Kanalmodulatoren mit einer überraschenden Selektivität für calciumabhängige Kalium-Kanäle großer Leitfähigkeit (BK(Ca)-Kanäle), insbesondere der Kalium-Kanäle des zentralen Nervensystems. Gleichzeitig zeichnen sie sich durch das Fehlen einer signifikanten Calcium-Kanalaktivität aus.

Aufgrund ihrer pharmakologischen Eigenschaften können sie für die Herstellung von Arzneimitteln zur Behandlung von zentral degenerativen Erkrankungen eingesetzt werden, wie z.B. bei Auftreten von Demenzen (Multiinfarktdemenz (MID), primär degenerativer Demenz (PDD), prä- und seniler Alzheimerscher Krankheit, HIV-Demenz und andere Demenzformen), Parkinsonscher Krankheit oder amyotrophischer Lateralsklerose sowie multipler Sklerose.

Weiterhin eignen sich die Wirkstoffe zur Behandlung von Hirnleistungsstörungen im Alter, des hirnorganischen Psychosyndroms (HOPS, Organic Brain Syndrom, OBS) und von altersbedingten Gedächtnisstörungen (age associated memory impairment, AAMI).

Sie sind geeignet zur Prophylaxe und Behandlung, und zur Bekämpfung der Folgen cerebraler Durchblutungsstörungen wie cerebraler Ischämien, Schlaganfällen, Schädel-Hirn-Traumata und von Subarachnoidalblutungen.

Sie sind wertvoll zur Behandlung von Depressionen und Psychosen, z.B. Schizophrenie. Außerdem eignen sie sich zur Behandlung von Störungen der neuroendokrinen Sekretion sowie der Neurotransmittersekretion und damit zusammenhängenden gesundheitlichen Störungen wie Manie, Alkoholismus, Drogenmißbrauch, Sucht oder krankhaftem Eßverhalten Weitere Anwendungsgebiete sind die Behandlung von Migräne, Schlafstörungen und von Neuropathien. Darüber hinaus sind sie als Schmerzmittel geeignet.

Die Wirkstoffe sind ferner geeignet zur Behandlung von Störungen des Immunsystems, insbesondere der T-Lymphocyten-Proliferation und zur Beeinflussung der glatten Muskulatur, insbesondere von Uterus, Harnblase und Bronchialtrakt und zur Behandlung damit zusammenhängender Krankheiten wie z.B. Asthma und urinärer Inkontinenz und zur Behandlung von Arrhythmie, Angina und Diabetes.

Die Erfindung betrifft außerdem neue Verbindungen der allgemeinen Formel (I) 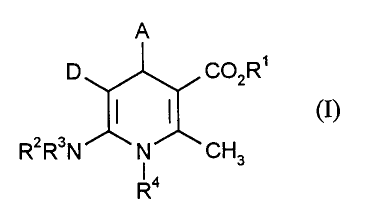
und deren Salze,
mit den in den folgenden Tabellen angegebenen Substituentenbedeutungen:

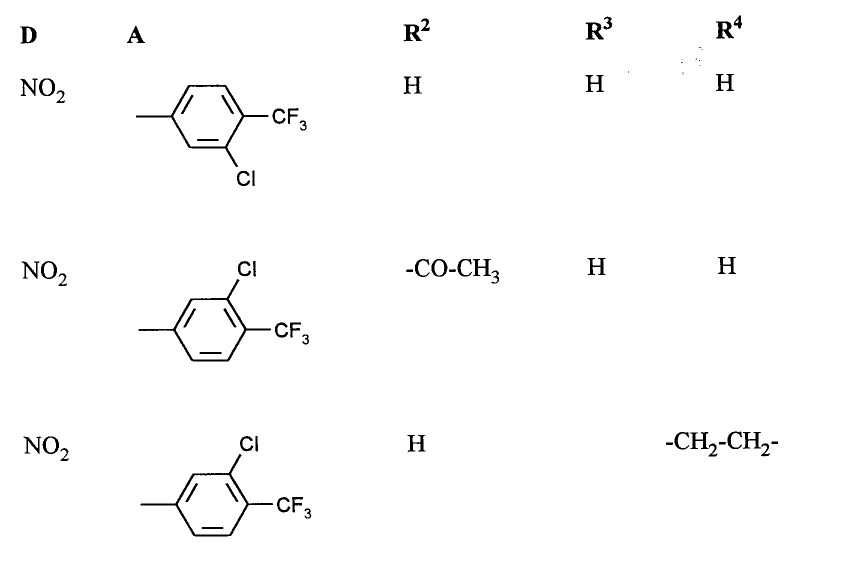

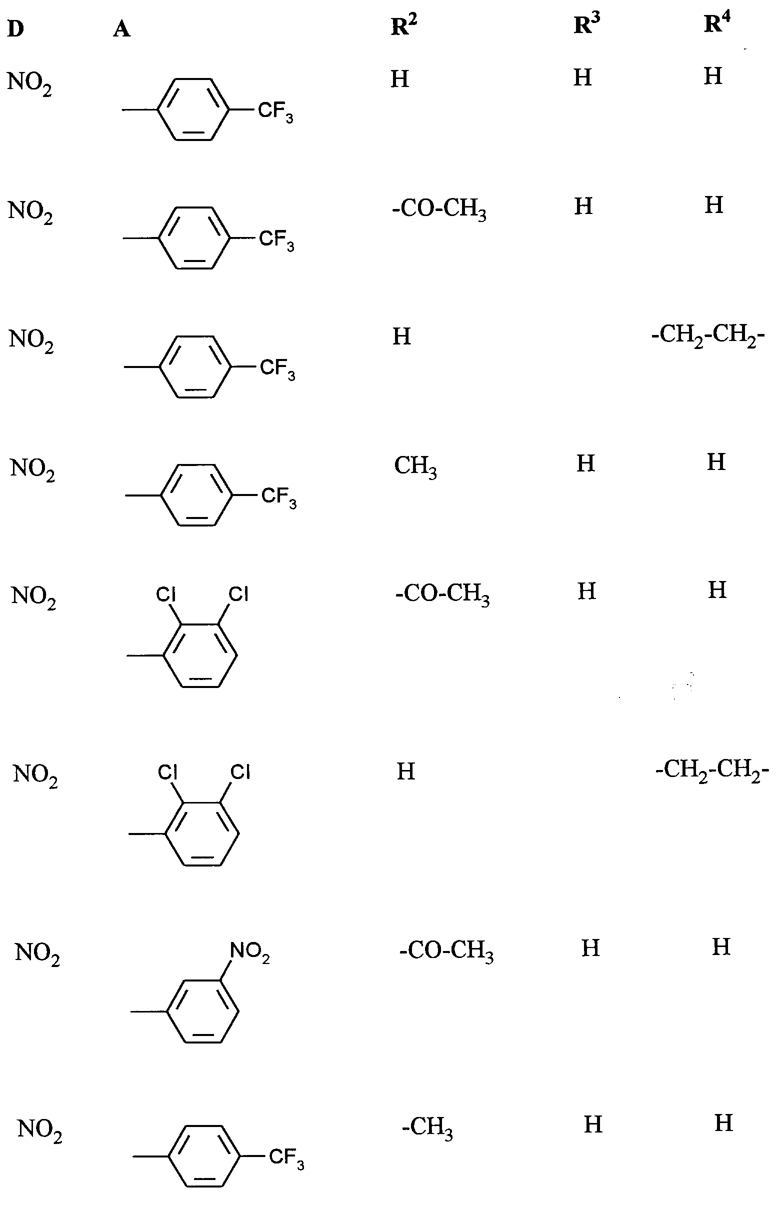

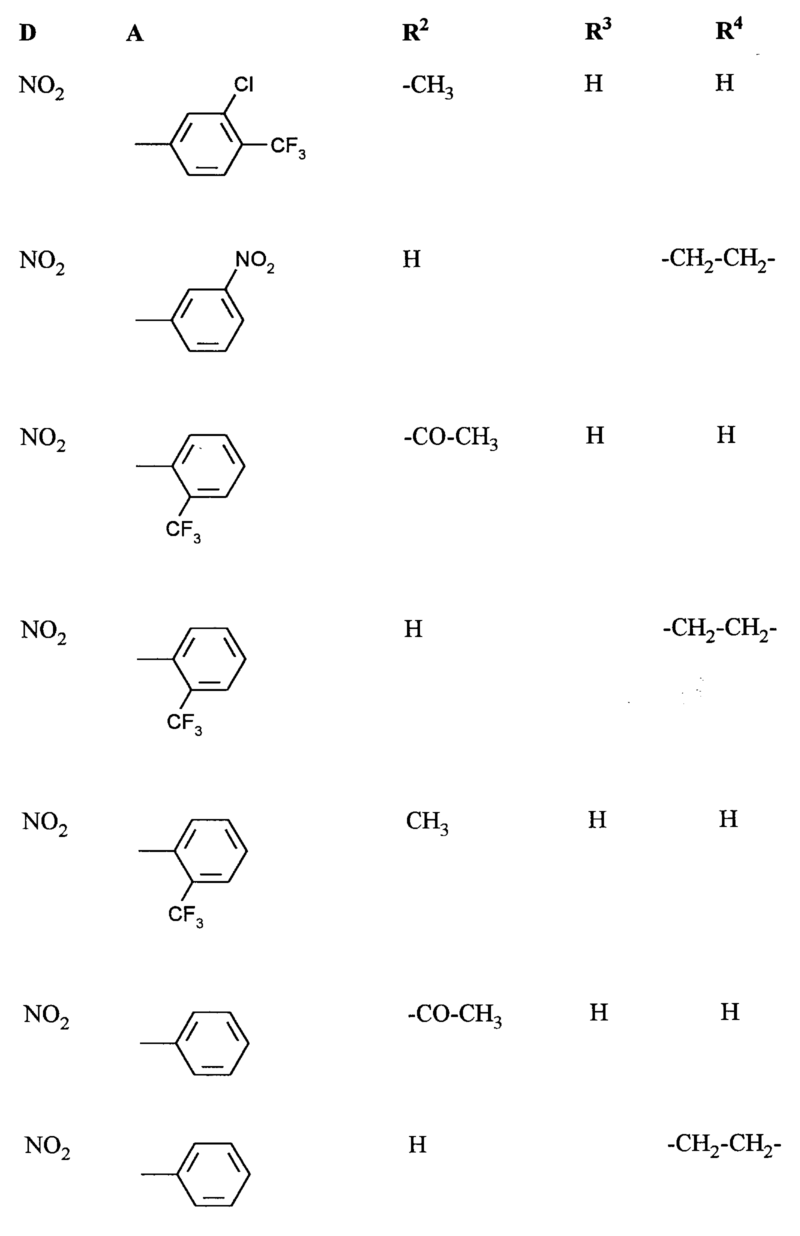

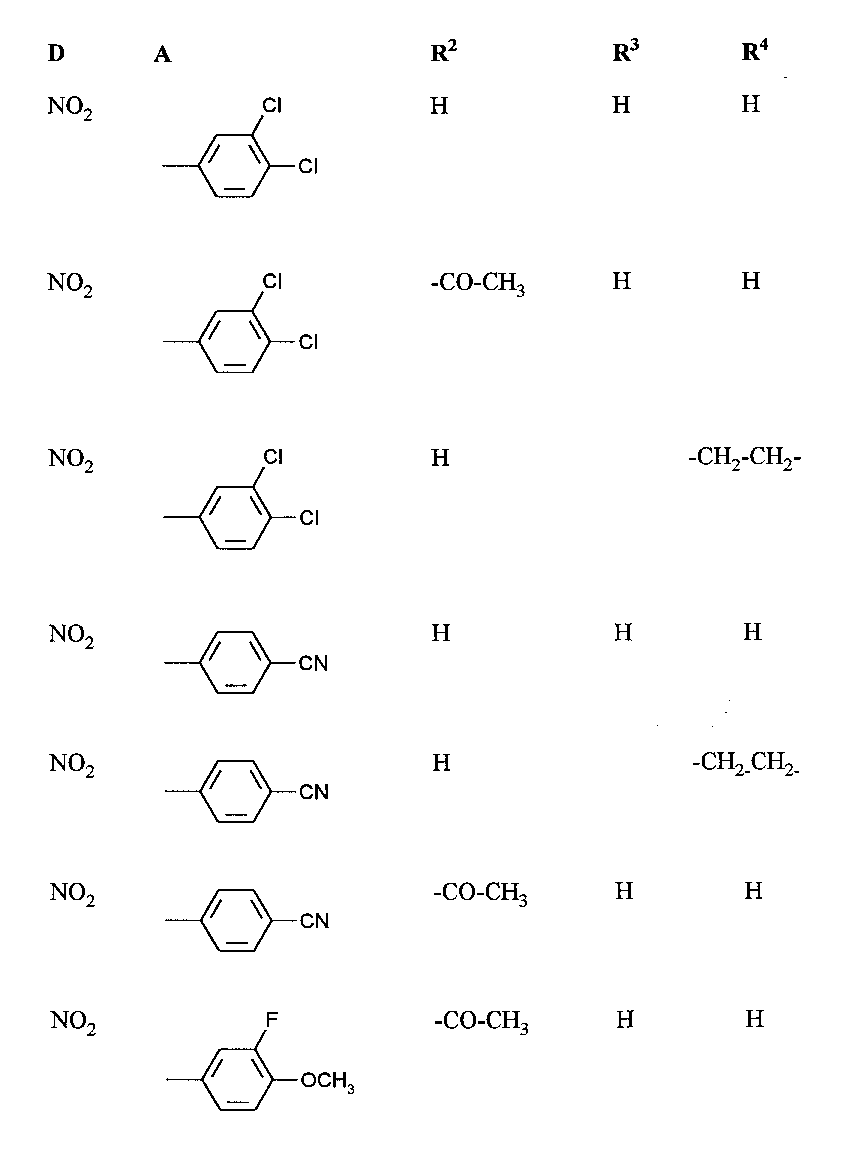

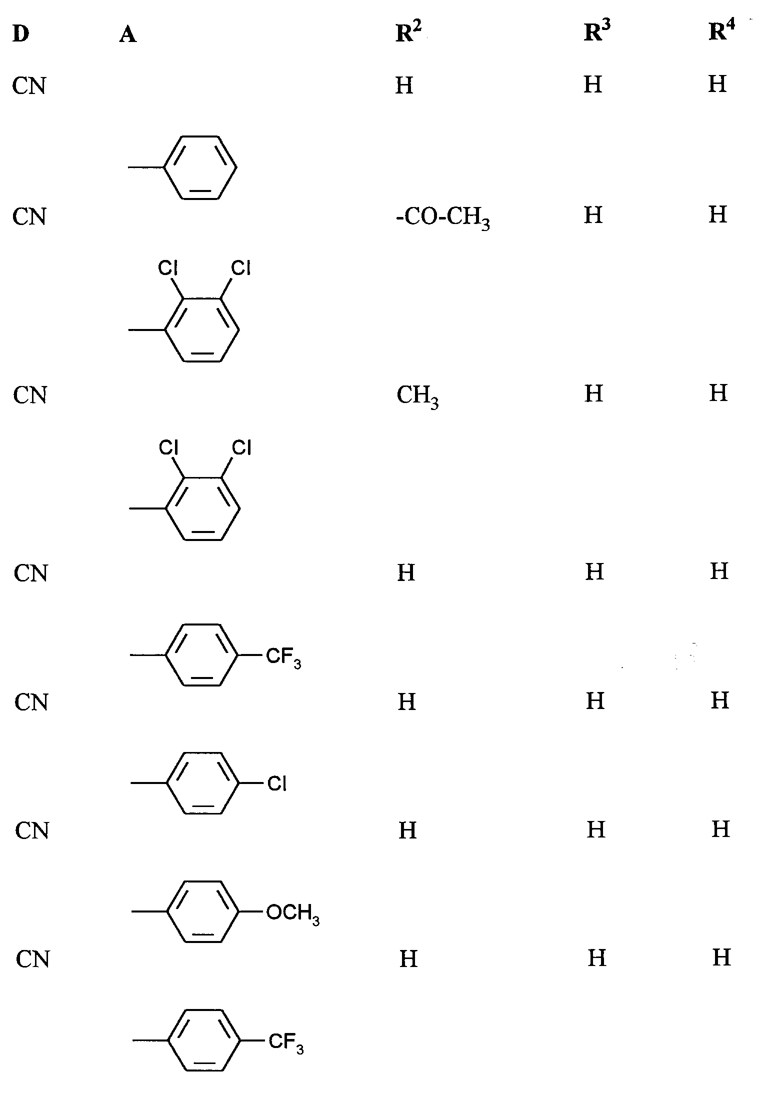

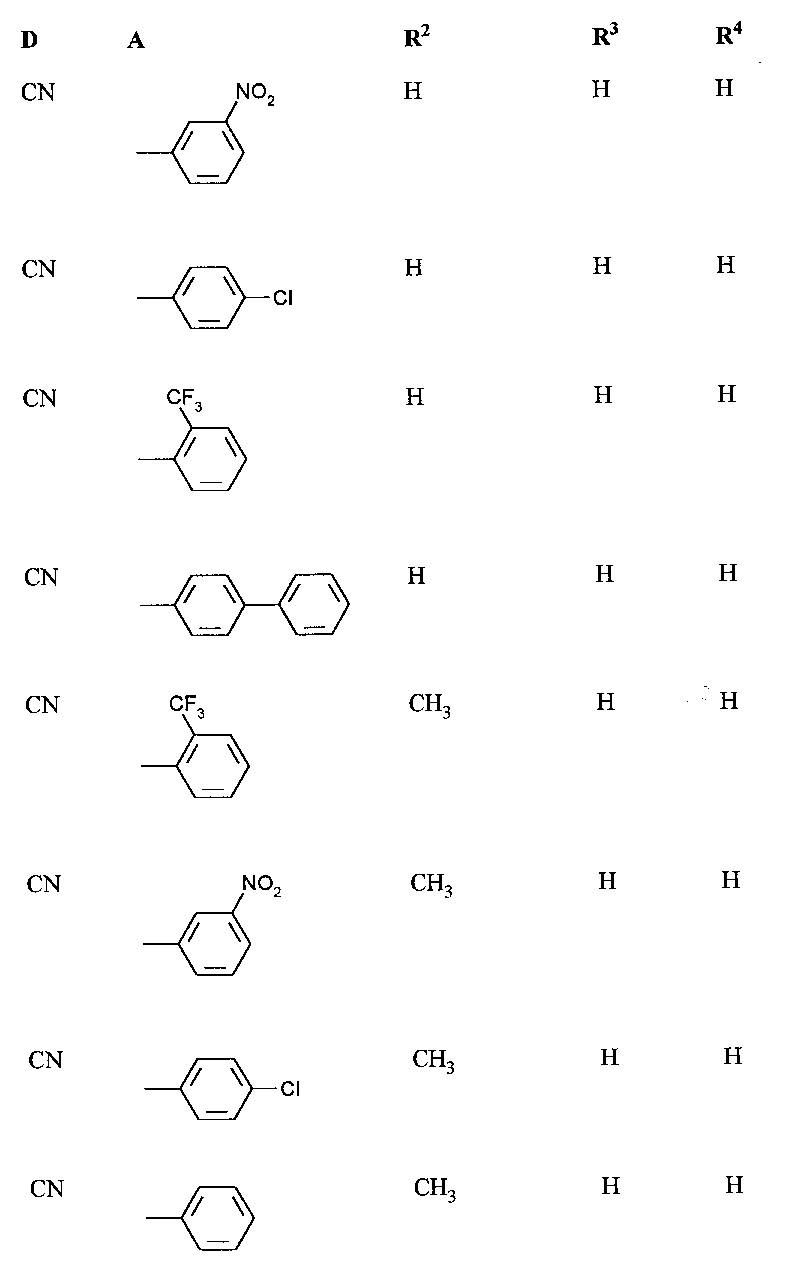

Die erfindungsgemäßen Verbindungen und die neuen Stoffe der allgemeinen Formeln (I) können hergestellt werden, indem man
Verbindungen der allgemeinen Formel (II) 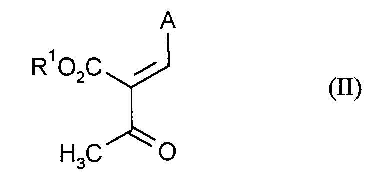
in welcher
- A und R¹: die oben angegebene Bedeutung haben
aber
- R¹: nicht für Wasserstoff steht,
im Fall D = NO₂ mit Verbindungen der allgemeinen Formel (III) 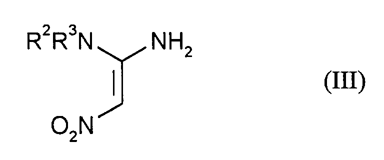
in welcher
- R² und R³: die oben angegebene Bedeutung haben,
und im Fall D = CN mit Verbindungen der allgemeinen Formel (IV) (R²/R³ = H) 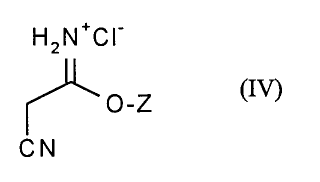
in welcher
- Z: für C₁-C₄-Alkyl steht,
in organischen Lösemitteln, gegebenenfalls in Anwesenheit eines Ammoniumsalzes, vorzugsweise Ammoniumacetat,
umsetzt,
im Fall R² und/oder R³ ≠ H mit der entsprechenden alkoholischen Aminlösung umsetzt, oder gegebenenfalls eine Alkylierung oder Acylierung, in organischen Lösemitteln und in Anwesenheit einer Base vornimmt,
und im Fall R¹ = H die Ester nach üblichen Methoden hydrolysiert.

Das erfindungsgemäße Verfahren kann durch folgendes Formelschema beispielhaft erläutert werden: 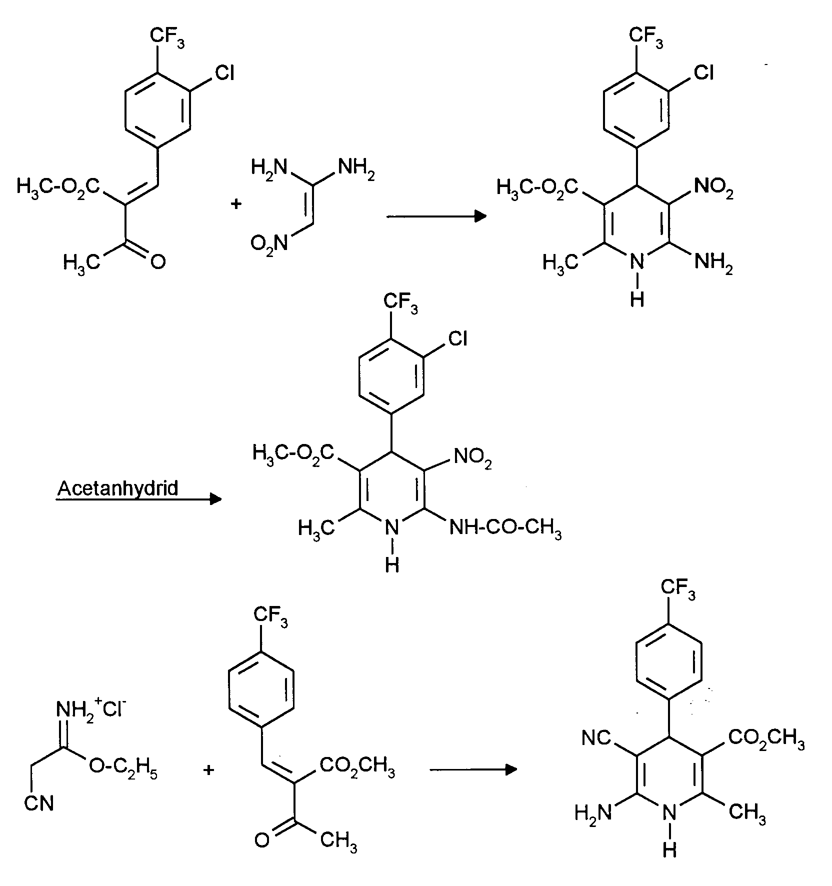 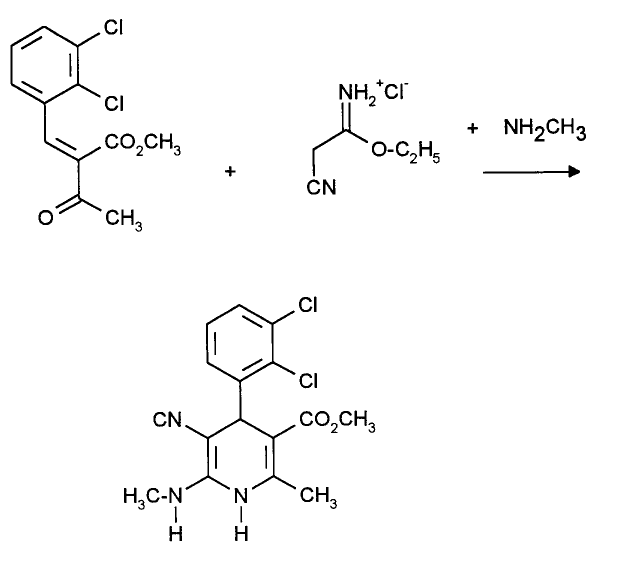
Als Lösemittel eignen sich hierbei alle inerten organischen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, Tetrahydrofuran, Glykoldimethylether, oder Diethylenglykoldimethylether, Acetonitril, oder Amide wie Hexamethylphosphorsäuretriamid oder Dimethylformamid, oder Essigsäure oder halogenierte Kohlenwasserstoffe wie Methylenchlorid, Tetrachlorkohlenstoff oder Kohlenwasserstoffe wie Benzol oder Toluol. Ebenso ist es möglich, Gemische der genannten Lösemittel zu verwenden. Besonders bevorzugt sind Isopropanol, Ethanol, Tetrahydrofuran, Methanol, Dioxan und Dimethylformamid.

Als Basen eignen sich im allgemeine Alkalihydride oder -alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt sind Piperidin, Dimethylaminopyridin, Pyridin, Natriumhydrid und Kalium-tert.butylat.

Bei der Durchführung der erfindungsgemäßen Verfahren ist das Verhältnis der an der Reaktion beteiligten Stoffe beliebig. Im allgemeinen arbeitet man jedoch bei äquimolaren Mengen der Reaktanden.

Die Reaktionstemperaturen können in einem größeren Bereich variiert werden. Im allgemeinen arbeitet man zwischen +10°C und +150° C, vorzugsweise zwischen +20°C und +100°C, insbesondere bei der Siedetemperatur des jeweiligen Lösemittels.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Als Lösemittel für die Alkylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dimethylformamid.

Als Basen eignen sich im allgemeinen Alkalihydride- oder alkoholate, wie beispielsweise Natriumhydrid oder Kalium-tert.butylat, oder cyclische Amine, wie beispielsweise Piperidin, Dimethylaminopyridin oder C₁-C₄-Alkylamine, wie beispielsweise Triethylamin. Bevorzugt ist Natriumhydrid..

Die Reaktionstemperaturen können in einem größeren Bereich variiert werden. Im allgemeinen arbeitet man zwischen +10°C und +150°C, vorzugsweise zwischen +20°C und +100°C, insbesondere bei Raumtemperatur.

Die Alkylierung wird in den oben aufgeführten Lösemitteln bei Temperaturen von 0°C bis +150° C, vorzugsweise bei Raumtemperaturen bis +100°C durchgeführt.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Die Base wird im allgemeinen in einer Menge von 1 mol bis 5 mol, bevorzugt von 1 mol bis 2 mol jeweils bezogen auf 1 mol der zu alkylierenden Verbindungen eingesetzt.

Als Basen für die Acylierung eignen sich organische Basen wie beispielsweise organische tertiäre Amine, Trialkyl(C₁-C₆)amine wie Triethylamin, oder Heterocyclen wie Pyridin, 4-(N,N-Dimethylamino)pyridin oder Methylpiperidin. Besonders bevorzugt ist Triethylamin.

Als Lösemittel für die Acylierung eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden oder das jeweilige Acylierungsmittel auch als Lösemittel einzusetzen. Bevorzugt sind Acetanhydrid und Pyridin.

Die Acylierung verläuft im allgemeinen in einem Temperaturbereich von 0°C bis +120°C, vorzugsweise bei +30°C bis +90°C und bei Normaldruck.

Die Verseifung der Carbonsäureester erfolgt nach üblichen Methoden, indem man die Ester in inerten Lösemitteln mit üblichen Basen behandelt.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Enantiomerenreine Formen erhält man z.B., indem man Diastereomerengemische der Verbindungen der allgemeinen Formel (I) nach üblicher Methode trennt, anschließend entweder direkt umestert oder zuerst die chiralen Carbonsäuren herstellt und dann durch Veresterung die enantiomerenreinen Dihydropyridine herstellt.

Die Trennung der Diastereomeren erfolgt im allgemeinen entweder durch fraktionierte Kristallisation, durch Säulenchromatographie oder durch Craig-Verteilung. Welches das optimale Verfahren ist, muß von Fall zu Fall entschieden werden, manchmal ist es auch zweckmäßig, Kombinationen der einzelnen Verfahren zu benutzen. Besonders geeignet ist die Trennung durch Kristallisation oder Craig-Verteilung bzw. eine Kombination beider Verfahren.

Die enantiomerenreinen Verbindungen sind auch zugänglich durch Chromatographie der racemischen Ester auf chiralen Phasen.

Die Verbindungen der allgemeinen Formel (II), (III) und (IV) sind bekannt oder können nach üblichen Methoden hergestellt werden.

Die erfindungsgemäßen Verbindungen der allgemeinen Formel (I) zeigen ein nicht wertvolles vorhersehbares Wirkspektrum, insbesondere aufgrund ihrer Selektivität auf calciumabhängige Kalium-Kanäle mit großer Leitfähigkeit.

### ⁸⁶Rubidium-Efflux aus C6-BU1-Glioma-Zellen

Die Versuche wurden mit geringfügigen Veränderungen entsprechend der von Tas et al. (Neurosci. Lett. 94, 279-284, (1988)) beschriebenen Methode durchgeführt. Dazu werden Ratten C6-BU1-Glioma-Zellkulturen verwendet. Aus den Daten wird die durch Ionomycin hervorgerufene Erhöhung des ⁸⁶-Rubidium-Effluxes über den Basalefflux berechnet und als 100 % gesetzt. Die Stimulationen in Gegenwart von Prüfsubstanzen werden dann auf diesen Wert bezogen

Zur vorliegenden Erfindung gehören auch pharmazeutische Zubereitungen, die neben inerten, nicht-toxischen, pharmazeutisch geeigneten Hilfs- und Trägerstoffen eine oder mehrere Verbindungen der allgemeinen Formel (I) enthalten, oder die aus einem oder mehreren Wirkstoffen der Formel (I) bestehen, sowie Verfahren zur Herstellung dieser Zubereitungen.

Die Wirkstoffe der Formel (I) sollen in diesen Zubereitungen in einer Konzentration von 0,1 bis 99,5 Gew.-%, bevorzugt von 0,5 bis 95 Gew.-% der Gesamtmischung vorhanden sein.

Neben den Wirkstoffen der Formel (I) können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, den oder die Wirkstoffe der Formel (I) in Gesamtmengen von etwa 0,01 bis etwa 100 mg/kg, bevorzugt in Gesamtmengen von etwa 1 mg/kg bis 50 mg/kg Körpergewicht je 24 Stunden, gegebenenfalls in Form mehrerer Einzelgaben, zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament, der Art und Schwere der Erkrankung, der Art der Zubereitung und Applikation, sowie dem Zeitpunkt bzw. Intervall, zu welchem die Verabreichung erfolgt.

### Ausgangsverbindungen

### Beispiel I

### 2-Acetyl-3-(3-chlor-4-trifluormethylphenyl)acrylsäuremethylester

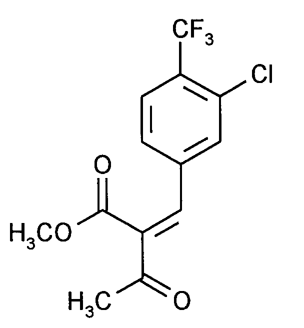

32,3 g (155 mmol) 3-Chlor-4-trifluormethylbenzaldehyd und 18,0 g (155 mmol) Acetessigsäuremethylester werden in 200 ml Methylenchlorid gelöst und mit 1,2 ml Piperidin sowie mit 1 ml Eisessig versetzt. Man erhitzt 5 h am Wasserabscheider. Dann wird mit Wasser gewaschen, über MgSO₄ getrocknet und eingeengt. Umkristallisation erfolgt zweimal aus Methanol. Man erhält 25,6 g (54% d.Th.) der Titelverbindung.

### Beispiel II

### 1-N-Methylamino-2-nitro-ethylen-1-amin

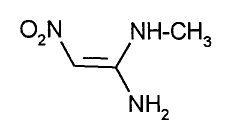

25,0 g (0,15 mol) 1,1-Bis(methylthio)-2-nitroethen werden in 300 ml Ethanol gelöst und mit 11,9 g (0,18 mol) Methylaminhydrochlorid versetzt. Man erhitzt zum Rückfluß und versetzt alle 30 min mit 4 Portionen von insgesamt 20,2 g (0,18 mol) Kalium-tert.butylat. Dann hält man 1 h bei Rückfluß (DC-Kontrolle: Acetessigsäureethylester / Petrolether 3:1). Man engt ein, nimmt in Methlyenchlorid auf, wäscht mit Wasser und trocknet über Natriumsulfat. Dann wird eingeengt und der Rückstand über Kieselgel filtriert (Methylenchlorid). Man kristallisiert aus Ethanol. Der isolierte Feststoff wird daraufhin in 200 ml Butanol gelöst und in die Lösung unter Rückfluß 2 h Ammoniak eingeleitet (DC-Kontrolle). Man destilliert ab und kristallisiert aus Ethanol. Ausbeute: 2,9 g (16% d.Th.)

### Herstellungsbeispiele

### Beispiel 1

### 6-Amino-4-(3-chlor-4-trifluormethylphenyl)-1,4-dihydro-2-methyl-5-nitro-3-carbonsäure-methylester

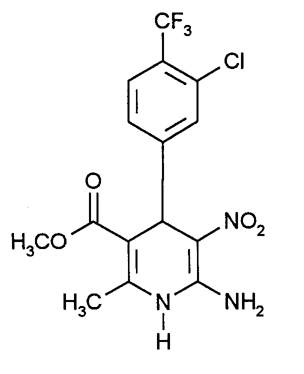

15,3 g (50 mmol) der Verbindung aus Beispiel I und 5,2 g (50 mmol) 2-Nitro-1,1-ethendiamin werden in 80 ml Ethanol gelöst und 12 h unter Rückfluß gehalten. Nach Abkühlen wird der entstandene Feststoff abgesaugt und mit Ethanol gewaschen. Man erhält 13,0 g (66% d. Th.) der Titelverbindung.
Smp.: 250°C
In Analogie zur Vorschrift des Beispiels 1 werden die in der Tabelle 1 aufgeführten Verbindungen hergestellt:

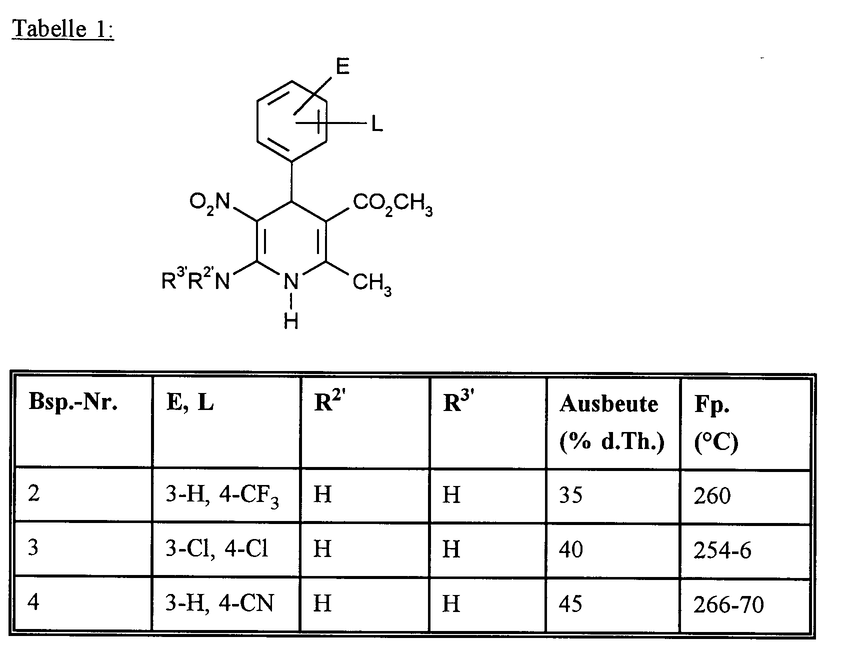

### Beispiel 5

### 6-Acetylamino-4-(3-chlor-4-trifluormethylphenyl)-1,4-dihydro-2-methyl-3-nitro-5-carbonsäure-methylester

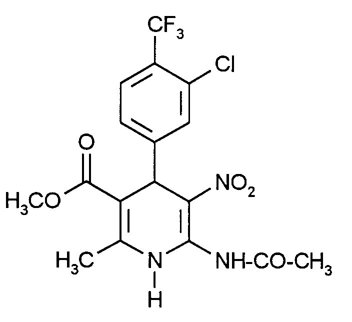

4,0 g (10,2 mmol) der Verbindung aus Beispiel 1 werden in 40 ml Acetanhydrid gelöst und 12 h unter Rückfluß erhitzt. Dann destilliert man das Acetanhydrid unter reduziertem Druck ab, löst den Rückstand in Methylenchlorid und wäscht mit ges. wäßriger NaHCO₃-Lösung. Die organische Phase wird getrocknet (MgSO₄), eingeengt und der Rückstand chromatographisch an Kieselgel gereinigt (Toluol/AcOEt/iPrOH 100+10+1). Das eingeengte Eluat wird aus Ethanol umkristallisiert. Man erhält 0,5 g (11% d.Th.) der Titelverbindung.
Smp.: 152°C
In Analogie zur Vorschrift des Beispiels 5 werden die in der Tabelle 2 aufgeführten Verbindungen hergestellt:

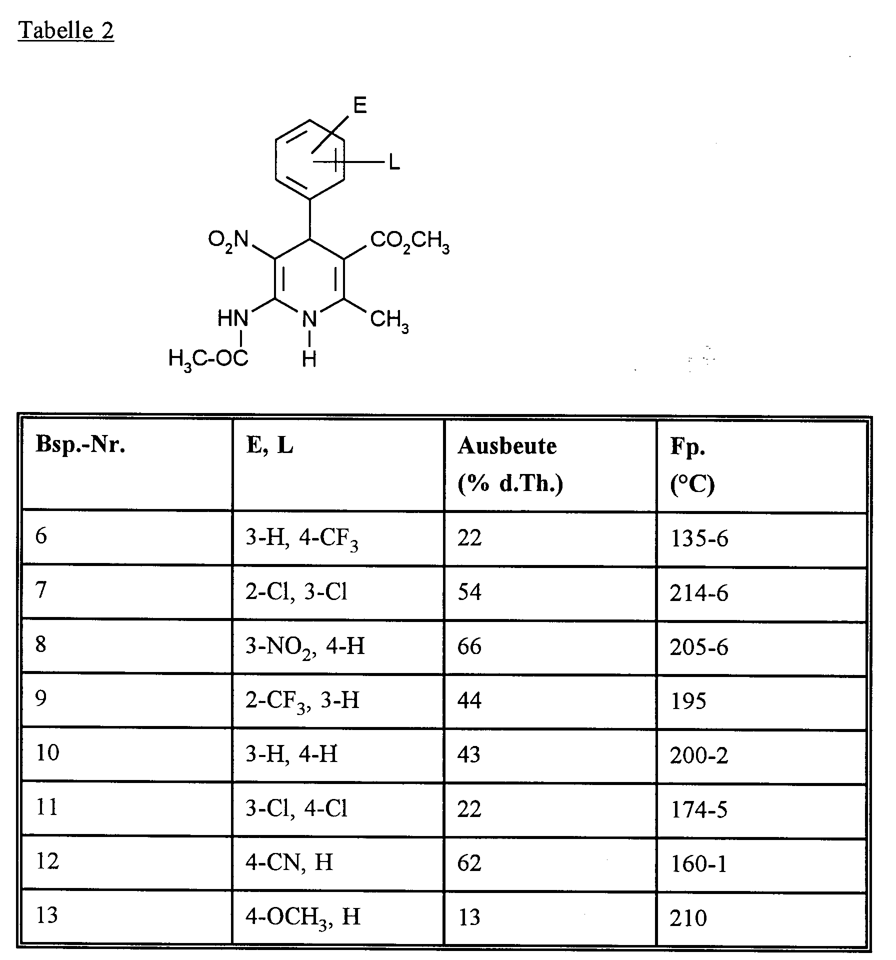

### Beispiel 14

### 4-(2,3-Dichlorphenyl)-3-methoxycarbonyl-2-methyl-3-nitro-1,7-diaza[4.3.0]bicyclonona-2,4-dien

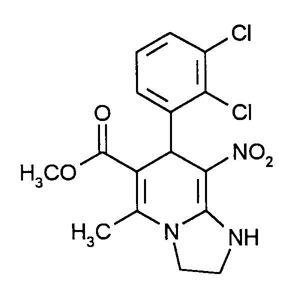

5,7 g (20 mmol) 2,3-Dichlorbenzylidenacetessigsäuremethylester und 2,6 g (20 mmol) 2-Nitromethylen-1,3-diazolidin werden in 30 ml Ethanol gelöst und 18 h auf 80°C erhitzt. Nach Abkühlen saugt man den entstandenen Feststoff ab und wäscht ihn mit Ethanol. Dann suspendiert man in Toluol, versetzt mit 20 mg p-Toluolsulfonsäure und hält 18 h am Wasserabscheider unter Rückfluß. Der beim Abkühlen kristallisierende Feststoff wird abgesaugt. Man erhält 6,1 g (79% d.Th.) der Titelverbindung.
Smp.: 225°C
In Analogie zur Vorschrift des Beispiels 14 werden die in Tabelle 3 aufgeführten Verbindungen hergestellt

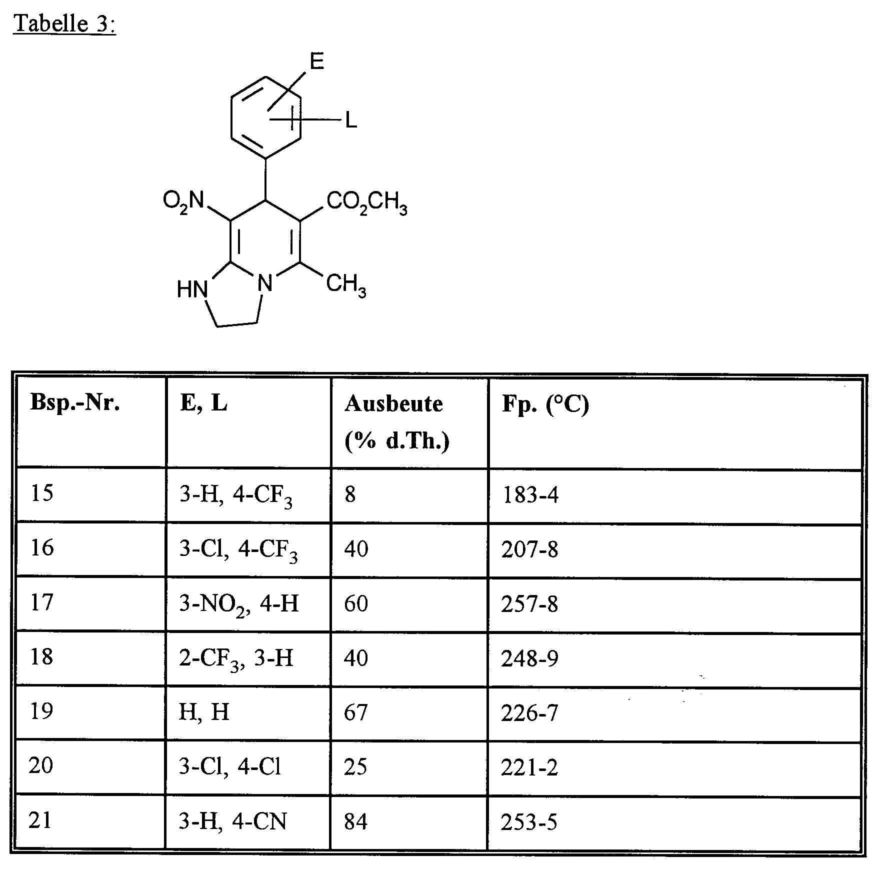

### Beispiel 22

### 1,4-Dihydro-2-methyl-6-methylamino-4-(3-chlor-4-trifluormethylphenyl)-5-nitro-3-carbonsäuremethylester

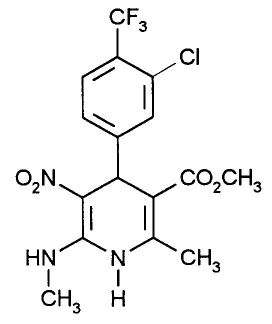

2,9 g (25 mmol) 1-N-Methylamino-2-nitroethylen-1-amin und 6,7 g (25mmol) 2-Acetyl-3-(3-chlor-4-trifluormethylphenyl)acrylsäuremethylester werden in 100 ml Ethanol gelöst und über Nacht zum Rückfluß erhitzt. Man engt die Reaktionsmischung ein und chromatographiert zweimal über Kieselgel (1: Methylenchlorid: AcOEt (Gradient), 2: Methylenchlorid : MeOH 100+1). Man erhält nach Umkristallisation aus EtOH/Acetonitril 10+1 1,2 g (21,6% d.Th.) der Titelverbindung.

In Analogie zur Vorschrift des Beispiels 22 werden die in Tabelle 4 aufgeführten Verbindungen hergestellt:

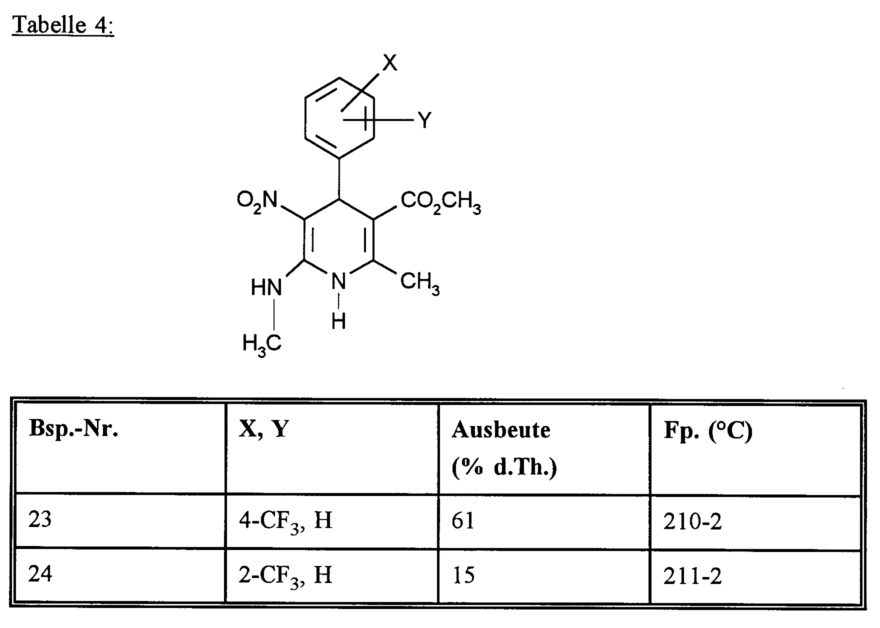

### Beispiel 25

### 6-Amino-5-cyan-2-methyl-4-(4-trifluormethylphenyl)-1,4-dihydropyridin-3-carbonsäuremethylester

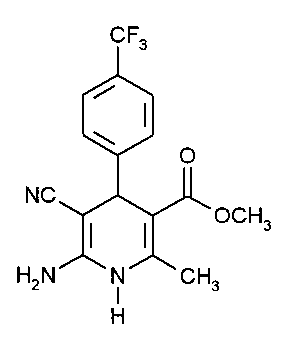

13,6 g (50 mmol) 2-Acetyl-3-(4-trifluormethylphenyl)acrylsäuremethylester, 7,45 g (50 mmol) Cyanacetimidsäureethylesterhydrochlorid und 15 g (190 mmol) Ammoniumacetat werden in 100 ml Methanol 1 h zum Rückfluß erhitzt. Nach dem Einengen wird der Rückstand zwischen Eiswasser und Essigsäureethylester verteilt. Die organische Phase wird 2 mal mit verdünnter wäßriger NaHCO₃-Lösung und 1 mal mit Wasser gewaschen, über Na₂CO₃ getrocknet und im Vakuum eingeengt. Kristallisation des Rückstands (18,4 g) aus Methanol liefert 6,3 g (37% d. Th.) farblose Kristalle der Titelverbindung.
Smp.: 210-214°C
In Analogie zur Vorschrift des Beispiels 25 werden die in Tabelle 5 dargestellten Verbindungen hergestellt:

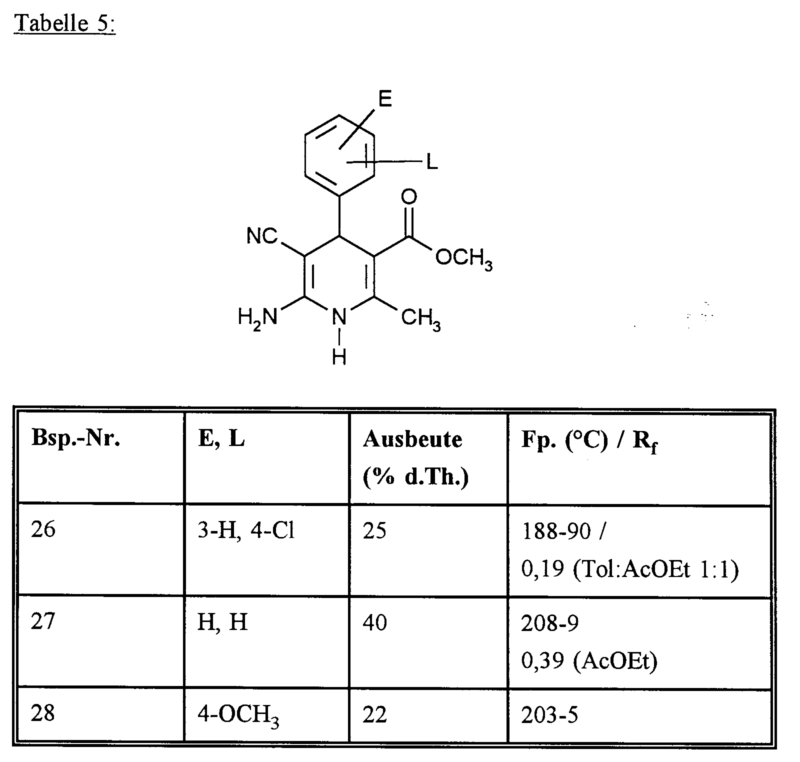

### Beispiel 29

### 6-Acetamido-5-cyan-4-(2,3-dichlorphenyl)-2-methyl-1,4-dihydropyridin-3-carbonsäuremethylester

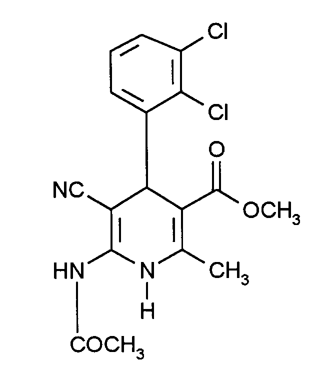

6,7 g (20 mmol) 6-Amino-5-cyan-4-(2,3-dichlorphenyl)-2-methyl-1,4-dihydropyridin-3-carbonsäuremethylester und 33,5 ml (350mmol) Acetanhydrid werden 30 min zum Rückfluß erhitzt. Überschüssiges Acetanhydrid wird anschließend durch Zugabe von Methanol unter Rühren bei 25°C zu Methylacetat umgesetzt. Die Reaktionslösung wird im Vakuum eingedampft und 2 mal mit Toluol im Vakuum abgezogen. Der Rückstand wird daraufhin mit 50 ml Toluol aufgekocht. Die abgeschiedenen Kristalle werden abfiltriert und mit Toluol gewaschen.
Ausbeute: 3,6 g (50% d.Th.)
Smp.: 224°C (Zers.)
In Analogie zur Vorschrift des Beispiels 29 werden die in Tabelle 6 aufgeführten Verbindungen hergestellt:

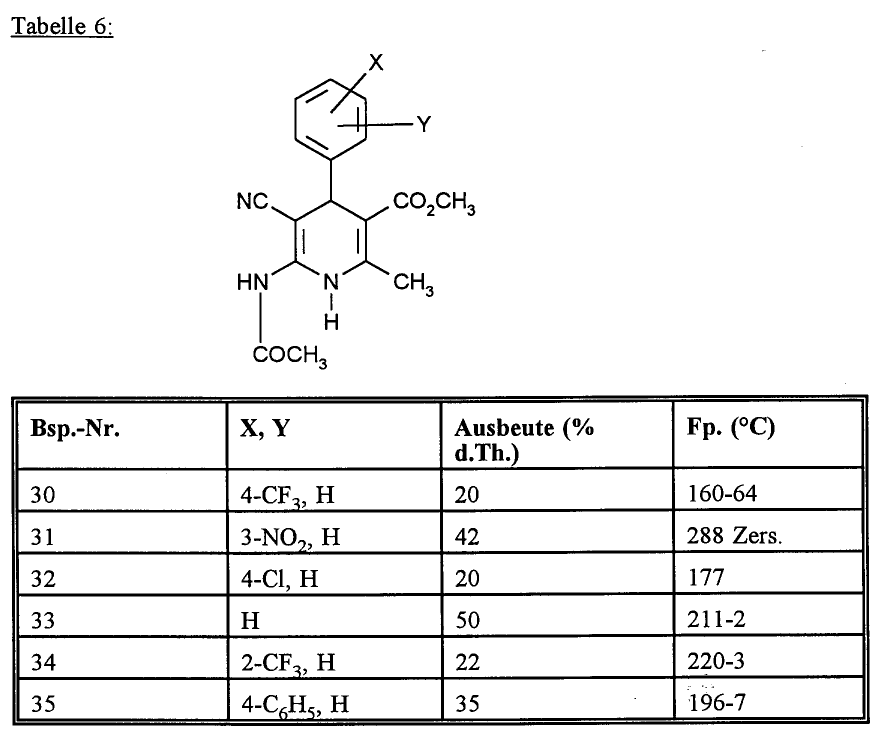

### Beispiel 36

### 5-Cyan-4-(2,3-dichlorphenyl)-2-methyl-6-N-methylamino-1,4-dihydropyridin-3-carbonsäuremethylester

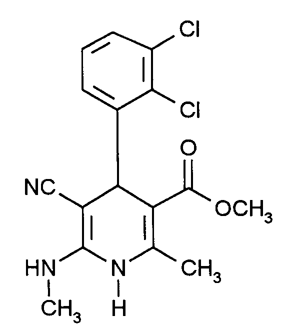

2,8 g (10 mmol) 2-Acetyl-3-(2,3-dichlorphenyl)acrylsäuremethylester und 1,5 g (10 mmol) Cyanacetimidsäureethylester-hydrochlorid werden mit 5 ml (33%iger) ethanolischer Methylaminlösung (40 mmol) versetzt. Das Gemisch erwärmt sich auf 46°C. Nach Abkühlen auf 30°C werden 2,3 ml (40 mmol) Eisessig und anschließend 20 ml Methanol zugegeben. Nach 5 h Erhitzen zum Rückfluß wird die Reaktionslösung mit Eiswasser versetzt und mit Acetessigsäureethylester extrahiert. Trocknen der organischen Phase (Na₂SO₄) und Einengen unter reduziertem Druck liefert 3,9 g amorphen Rückstand, der an 100 g Kieselgel mit Toluol/Acetessigsäureethylester (Gradient) chromatographiert wird.
Ausbeute: 0,5 g (10% d.Th.) Kristalle
Smp.: 234-239°C
In Analogie zur Vorschrift des Beispiels 36 werden die in Tabelle 7 aufgeführten Verbindungen hergestellt:

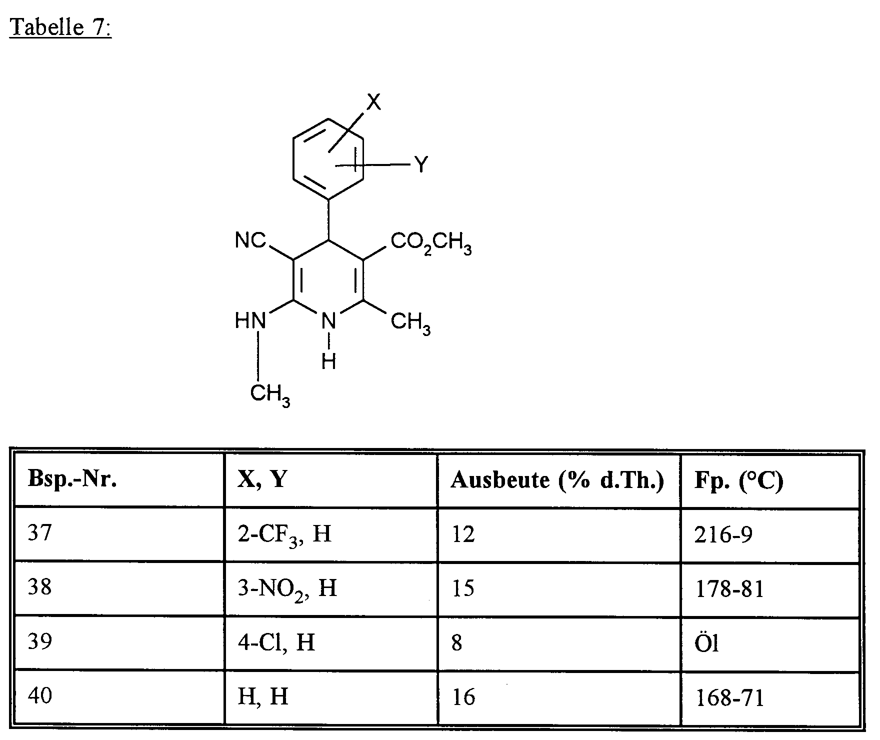

## Patentansprüche

1. Verwendung von 6-Amino-dihydropyridinen der allgemeinen Formel (I) 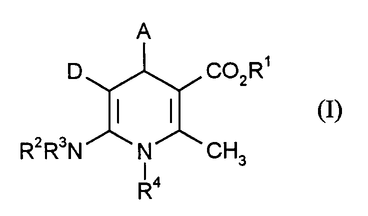 in welcher
A für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
D für Cyano oder Nitro steht,
R¹ für Wasserstoff oder für geradkettiges oder verzweigtes Alkyl mit bis zu 8 Kohlenstoffatomen steht,
R² und R³ gleich oder verschieden sind und
für Wasserstoff oder für geradkettiges oder verzweigtes Alkyl oder Acyl mit jeweils bis zu 6 Kohlenstoffatomen stehen,
R⁴ für Wasserstoff steht,
oder
R³ und R⁴ zusammen für einen Rest der Formel -(CH₂)ₙ stehen,
worin
n eine Zahl 2, 3 oder 4 bedeutet,
und deren physiologisch unbedenklichen Salze, zur Herstellung von Arzneimitteln mit selektiv-modulierender Wirkung auf Kaliumkanäle zur Behandlung des zentralen Nervensystems.

2. Neue 6-Acylamino-dihydropyridine der allgemeinen Formel (I) 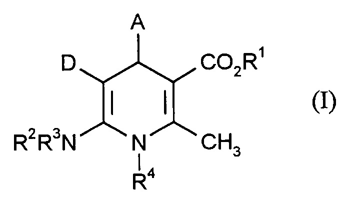 in welcher
A für Aryl mit 6 bis 10 Kohlenstoffatomen oder Pyridyl steht, die gegebenenfalls bis zu 3-fach gleich oder verschieden durch Nitro, Cyano, Phenyl, Halogen, Trifluormethyl oder durch geradkettiges oder verzweigtes Alkylthio oder Alkoxy mit jeweils bis zu 6 Kohlenstoffatomen substituiert sind,
D für Cyano oder Nitro steht,
R¹ für Wasserstoff oder für geradkettiges oder verzweigtes Alkyl mit bis zu 8 Kohlenstoffatomen steht,
R² und R³ gleich oder verschieden sind und für Wasserstoff oder für geradkettiges oder verzweigtes Alkyl oder Acyl mit jeweils bis zu 6 Kohlenstoffatomen stehen, wobei mindestens einer der Substituenten eine Acylgruppe bedeutet,
R⁴ für Wasserstoff steht
oder
R³ und R⁴ zusammen für einen Rest der Formel -(CH₂)ₙ stehen,
worin
n eine Zahl 2 oder 3 bedeutet,
und deren physiologisch unbedenklichen Salze, zur Herstellung von Arzneimitteln mit selektiv-modulierender Wirkung auf Kaliumkanäle zur Behandlung des zentralen Nervensystems.

3. Neue Verbindungen der allgemeinen Formel (I) gemäß Anspruch 1 ausgewählt aus der Gruppe der 40 Ausführungsbeispiele.

4. Verfahren zur Herstellung von Verbindungen der allgemeinen Formel (I) gemäß Anspruch 1, dadurch gekennzeichnet, daß man Verbindungen der allgemeinen Formel (II) 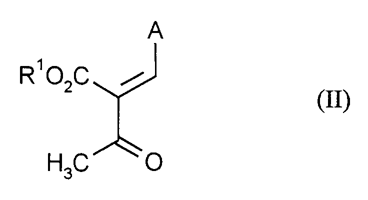 in welcher
A und R¹ die oben angegebene Bedeutung haben
aber
R¹ nicht für Wasserstoff steht,
im Fall D = NO₂ mit Verbindungen der allgemeinen Formel (III) 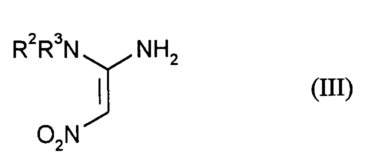 in welcher
R² und R³ die oben angegebene Bedeutunmg haben,
und im Fall D = CN mit Verbindungen der allgemeinen Formel (IV) (R²/R³=H) 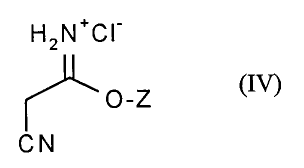 in welcher
Z für C₁-C₄-Alkyl steht,
in organischen Lösemitteln, gegebenenfalls in Anwesenheit einer Base, und einem Ammoniumsalz, vorzugsweise Ammoniumacetat,
umsetzt,
im Fall R² und/oder R³ ≠ H mit der entsprechenden alkoholischen Aminlösung umsetzt, oder eine Alkylierung oder Acylierung, gegebenenfalls in organischen Lösemitteln und in Anwesenheit einer Base vornimmt,
und im Fall R¹ = H die Ester nach üblichen Methoden hydrolysiert.

5. Verbindungen der allgemeinen Formel (I) 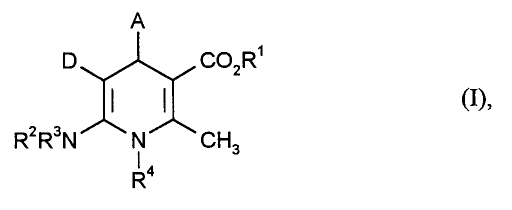 in welcher
A für Phenyl oder Pyridyl steht, die gegebenenfalls bis zu zweifach gleich oder verschieden durch Nitro, Cyano, Phenyl, Fluor, Chlor, Trifluormethyl, Methoxy oder Methylthio substituiert sind,
D für Cyano oder Nitro steht,
R¹ für geradkettiges oder verzweigtes Alkyl mit bis zu 4 Kohlenstoffatomen steht,
R² und R³ gleich oder verschieden sind und für Wasserstoff, geradkettiges oder verzweigtes Alkyl oder Acyl mit jeweils bis zu 4 Kohlenstoffatomen stehen, wobei mindestens einer der Substituenten Acyl bedeutet,
R⁴ für Wasserstoff steht,
oder
R³ und R⁴ zusammen für einen Ethylen- oder Propylenrest stehen,
und deren Salze.

6. Arzneimittel zur selektiven Behandlung von Kaliumkanälen im zentralen Nervensystem enthaltend mindestens eine Verbindung der allgemeinen Formel (I) gemäß Anspruch 1.

7. Verfahren zur Herstellung von Arzneimitteln, dadurch gekennzeichnet, daß man mindestens eine Verbindung der allgemeinen Formel (I) gemäß Anspruch 1 gegebenenfalls mit üblichen Hilfsstoffen in eine geeignete Applikationsform überführt.
